# EUROPÄISCHE PATENTANMELDUNG

(11) **EP 4 748 331 A1**
(43) Veröffentlichungstag der Anmeldung: **27.05.2026**
(21) Anmeldenummer: 25217627.6
(22) Anmeldetag: 21.11.2025
(51) Int. Cl.: A61B 18/14, A61B 34/30, A61B 17/29

(54) **CHIRURGISCHES MAULTEILWERKZEUG, CHIRURGISCHES INSTRUMENT UND ROBOTISCHES CHIRURGISCHES INSTRUMENTENSYSTEM**

(30) Priorität: 22.11.2024 DE 102024134411
(71) Anmelder: Karl Storz SE & Co. KG, 78532 Tuttlingen (DE)
(72) Erfinder: GRÜNER, Sven Axel, 78532 Tuttlingen (DE)

(57) **Zusammenfassung**

Die vorliegende Erfindung stellt ein bipolares chirurgisches Maulteilwerkzeug (8) und ein damit geschaffenes bipolares chirurgisches Instrument (50) bereit. Das Maulteilwerkzeug (8) hat zwei schwenkbar gelagerte Branchen (2, 2'), die ein elektrisch leitendes Material aufweisen, und ein Kraftübertragungsmittel (9), das mit den Branchen (2, 2') in Wirkverbindung steht, und ist dazu ausgebildet, durch Kopplung mit einem Instrumentenschaft (51) und mit einem Betätigungsmittel (52) das Instrument (50) bereitzustellen, wobei die Branchen (2, 2') distalseitig am Instrumentenschaft (51) angeordnet sind und das Kraftübertragungsmittel (9) in dem Instrumentenschaft (51) entlang einer Längsachse (L) beweglich angeordnet und mit dem Betätigungsmittel (52) proximalseitig am Instrumentenschaft (51) in Eingriff bringbar ist. Das Maulteilwerkzeug (8) hat einen Maulteilkern (1) aus einem elektrisch isolierenden Material und zwei durch eine Kernwand (1') voneinander getrennte Aussparungen (10, 10'), die jeweils eine Gelenkachse (A, A') bereitstellen, die die Aussparungen (10, 10') jeweils in einen Austritts- und einen Betätigungsschwenkbereich (13, 14) unterteilt. Dabei hat jede Branche (2, 2') zwischen einem Maulteil- und einem Betätigungsabschnitt (21, 22) einen Lagerungsabschnitt (23), an dem die Branche (2, 2') um die jeweilige Gelenkachse (A, A') schwenkbar in der Aussparung (10, 10') gelagert ist, wobei sich der Maulteilabschnitt (21) durch den Austrittsschwenkbereich (13) aus der Aussparung (10, 10') erstreckt und der Betätigungsabschnitt (22) in dem Betätigungsschwenkbereich (14) der Aussparung (10, 10') angeordnet ist. Das Maulteilwerkzeug (8) hat zwei Ansteuerungslaschen (3, 3'), die mit dem Kraftübertragungsmittel (9) verbunden sind und sich jeweils in den Betätigungsschwenkbereich (14) der Aussparungen (10, 10') erstrecken und dort mit dem Betätigungsabschnitt (22) der jeweiligen Branche (2, 2') in Eingriff stehen. Ferner wird ein robotisches chirurgisches Instrumentensystem offenbart.

## Beschreibung

Die Erfindung betrifft ein bipolares chirurgisches Maulteilwerkzeug für ein bipolares chirurgisches Instrument und ein bipolares chirurgisches Instrument mit einem solchen Maulteilwerkzeug. Ferner betrifft die Erfindung ein robotisches chirurgisches Instrumentensystem mit einem solchen bipolaren chirurgischen Instrument.

Aus dem Stand der Technik ist bekannt, bipolare chirurgische Instrumente mit einem Maulteilwerkzeug aus beweglichen Branchen, wie z. B. Präparier-, Fasszangen und Scheren anzuwenden, die einerseits je nach Form und Größe der Branchen eine Präparier-, Fass- oder Schneidefunktion und andererseits beim Anlegen von bipolarem Strom an den Branchen eine Koagulationsfunktion haben. Zur Anordnung des Werkzeugs an einem distalen Ende eines mit einem Betätigungsmittel (z. B. Griff, Aktuator oder Schnittstelle zu einem Robotersystem) verbundenen Schafts weist das Instrument einen Werkzeugeinsatz mit einer Maulteilmechanik zum Öffnen und Schließen der Branchen auf. Die Maulteilmechanik umfasst zumindest ein Gelenk, um dessen Gelenkachse zumindest eine der beiden Branchen bewegt werden kann. Man unterscheidet zwischen einseitig öffnenden Maulteilwerkzeugen, bei denen nur eine Branche bewegt wird, und beidseitig öffnenden Maulteilwerkzeugen, bei denen beide Branchen bewegt werden. In der Anwendung werden beidseitig öffnende Maulteilwerkzeuge bevorzugt. Unter Nutzung bipolarer Energie kann Strom kontrolliert zwischen den beiden Branchen fließen gelassen werden, sodass zwischen den Branchen befindliches Gewebe koaguliert wird. Da die Branchen auch als Elektroden fungieren, weisen sie ein elektrisch leitendes Material zur Bildung der Elektroden auf.

Um Kurzschlüsse zwischen den Branchen zu vermeiden, bestehen diese üblicherweise nicht vollständig aus dem elektrisch leitenden Material, sondern weisen zumindest im Bereich des Gelenks eine Isolationsschicht auf oder sind mehrteilig mit Isolierabschnitten aufgebaut. Solche Isolatoren aus elektrisch nichtleitenden Kunststoffen oder Keramiken haben meist eine Durchschlagsfestigkeit von 10 bis 50 kV/mm, sodass ein direkter Durchschlag durch den Isolator kaum auftritt, da hier bereits eine Schichtdicke von einem Zehntel Millimeter ausreicht, um mindestens 1.000 V voneinander zu trennen, und mechanische Festigkeit und Fertigung ohnehin meist schon Wandungsstärken von zumindest zwei Zehnteln Millimetern erfordern.

Allerdings ist eine andere Versagensart bei den bipolaren Instrumenten kritisch, nämlich ein Durchschlag entlang der Oberfläche des Isolators entlang einer sogenannten Kriechstrecke. Abhilfe hiergegen ist durch Vergrößerung des geometrischen Abstands zwischen den Branchen möglich, um die Kriechstrecke entlang der Isolatoroberfläche möglichst lang zu gestalten, sodass ein Durchschlag erst bei höheren Spannungen stattfindet. Weil aber in der Anwendung leitende Flüssigkeiten (Blut, Saline etc.) den Durchschlag begünstigen und diese sich insbesondere in abgedeckten Bereichen der Maulteilmechanik ansammeln, sind innenliegende Kriechstrecken noch problematischer. Abgesehen von dem Abstand beeinflusst der Werkstoff des Isolators die Anfälligkeit für die Bildung von Kriechstrecken. Hierbei wird die Kriechstromfestigkeit eines Isolatorwerkstoffs mit dem sogenannten CTI-Wert (*Comparative Tracking Index*) bestimmt. Dazu werden 50 Tropfen genormte Elektrolytlösung zwischen zwei Elektroden in vorbestimmtem Abstand auf die Isolatoroberfläche aufgetropft und mit den Elektroden an der Isolatoroberfläche die Spannung gemessen, bis zu der der Isolator kein Tracking zeigt bzw. bei der er leitfähig wird.

Für eine gute Kriechstromfestigkeit sind hohe CTI-Werte des Isolatorwerkstoffs vorteilhaft. Demgegenüber stehen allerdings mechanische Anforderungen an den Werkstoff, weshalb teilweise Kunststoffe als Isolator verwendet werden, die einen niedrigen CTI-Wert von 100 bis 150 aufweisen, wie z. B. PEEK. Denn Kunststoffe mit höherem CTI-Wert sind für die mechanischen Anforderungen häufig zu weich und Keramik ist zu spröde.

Aus DE 102 36 070 A1 ist ein Maulteilmechanismus einer medizinischen Zange bekannt. Das proximale Ende der beweglichen Maulteile weist an seinem proximalen Ende einen Gelenkarm auf, welcher in einer Führungsbahn geführt ist.

Die Offenlegungsschrift US 2006/0173452 A1 offenbart ein bipolares chirurgisches Instrument zum Verschließen von Gefäßen. Ein Anschlag stellt einen Mindestabstand zwischen den beiden beweglichen Maulteilen sicher.

Bislang erfüllt keines der bekannten bipolaren Maulteilinstrumente für chirurgische Anwendungen sämtliche daran gestellte Anforderungen, die eine hohe Anwendungsspannung, mechanische Festigkeit, Langlebigkeit, gute Reinigbarkeit und letztendlich geringe Bauteil- und Herstellungskosten umfassen.

Ausgehend von diesem Stand der Technik ist es Aufgabe der vorliegenden Erfindung, ein verbessertes Maulteilwerkzeug für ein bipolares chirurgisches Instrument bereitzustellen
Diese Aufgabe wird durch ein bipolares chirurgisches Maulteilwerkzeug mit den Merkmalen des Anspruchs 1 gelöst.

Die weitere Aufgabe, ein entsprechend verbessertes bipolares chirurgisches Instrument bereitzustellen, wird durch das bipolare chirurgische Instrument mit den Merkmalen des unabhängigen Anspruchs 15 gelöst.

Die noch weitere Aufgabe, ein entsprechend verbessertes robotisches chirurgisches Instrumentensystem mit einem bipolaren chirurgischen Instrument bereitzustellen, wird durch das robotische chirurgische Instrumentensystem mit den Merkmalen des unabhängigen Anspruchs 16 gelöst.

Weiterbildungen bzw. bevorzugte Ausführungsformen sind in den Unteransprüchen ausgeführt.

Gemäß einer ersten Ausführungsform weist ein erfindungsgemäßes bipolares chirurgisches Maulteilwerkzeug, das zur Bereitstellung eines bipolaren chirurgischen Instruments durch Kopplung mit einem Instrumentenschaft und einem Betätigungsmittel ausgebildet ist, zwei zum Öffnen und Schließen schwenkbar gelagerte Branchen und ein Kraftübertragungsmittel auf. Die beiden Branchen, die an einem distalen Ende des Instrumentenschafts angeordnet werden können, wenn sie das bipolare chirurgische Instrument bilden, weisen ein elektrisch leitendes Material auf, um Elektroden bereitzustellen. Sie stehen zur mechanischen und elektrischen Ansteuerung mit dem Kraftübertragungsmittel in Wirkverbindung. Das Kraftübertragungsmittel kann dabei bei der Ausbildung des Instrumentes in dem Instrumentenschaft entlang einer gemeinsamen Längsachse beweglich angeordnet werden und mit dem Betätigungsmittel an einem proximalen Ende des Instrumentenschafts in Eingriff gebracht sein. Erfindungsgemäß weist das Maulteilwerkzeug einen Maulteilkern auf, der aus einem elektrisch isolierenden Material besteht und zwei durch eine Kernwand voneinander getrennte Aussparungen aufweist. Beide Aussparungen sind dazu ausgebildet, jeweils eine Gelenkachse für die Branchen bereitzustellen, die die Aussparungen jeweils in einen Austrittsschwenkbereich und einen Betätigungsschwenkbereich unterteilt. Ferner weist jede Branche zwischen einem Maulteilabschnitt und einem Betätigungsabschnitt einen Lagerungsabschnitt auf, an dem jede Branche um die Gelenkachse schwenkbar in der jeweiligen Aussparung gelagert ist. Dabei erstreckt sich der Maulteilabschnitt durch den Austrittsschwenkbereich aus der Aussparung, und der Betätigungsabschnitt ist in dem Betätigungsschwenkbereich der Aussparung angeordnet. Außerdem weist das Maulteilwerkzeug zwei Ansteuerungslaschen auf, die als längliche, flache Verbindungsstücke die Wirkverbindung der Branchen mit dem Kraftübertragungsmittel bereitstellen. Dazu sind die Ansteuerungslaschen einenends mit dem Kraftübertragungsmittel verbunden und erstrecken sich anderenends jeweils in den Betätigungsschwenkbereich der Aussparungen und stehen mit dem darin angeordneten Betätigungsabschnitt der jeweiligen Branche in Eingriff.

Bei dem erfindungsgemäßen Maulteilwerkzeug sorgt der Maulteilkern nicht nur für die mechanische Lagerung der beiden beidseitig öffnenden Branchen, sondern auch für eine sichere elektrisch Isolation der beiden Branchen. Ferner sind die Funktionen zur mechanischen und elektrischen Ansteuerung beider Branchen in den Ansteuerungslaschen vereint. Dabei erreicht das Maulteilwerkzeug in dem für chirurgische Werkzeuge begrenzten Bauraum, insbesondere auch für Werkzeugdurchmesser von 5,5 mm oder weniger, z. B. 3,5 mm, eine hohe mechanische Stabilität im gemeinsamen Zusammenwirken der in dem Maulteilkern gelagerten und mit den Ansteuerungslaschen verbundenen Branchen.

Die durch den Maulteilkern bereitgestellte Mechanik hat günstige Kraftflüsse, die die Verwendung von elektrisch nichtleitenden Materialien mit hohen CTI-Werten ermöglichen, insbesondere auch Keramiken oder Kunststoffe wie beispielsweise Polybutylenterephthalat (PBT) mit einem CTI von 500, Polyethylen (PE-LD, PE-HD), Polyesterharz, Polytetrafluorethylen (PTFE), Polyvinylidenfluorid (PVDF) jeweils mit einem CTI von 600.

Unter einem "Kraftübertragungsmittel" werden langgestreckte Vorrichtungen verstanden, die zur Übertragung einer von dem proximalen Betätigungsmittel erzeugten translatorischen Kraft- bzw. Bewegung durch den Instrumentenschaft auf die distale Maulteilmechanik geeignet sind, wie beispielsweise starre oder flexible Zug-Druckstangen. Bei einem flexiblen Kraftübertragungsmittel bezieht sich die Längsachse auf eine gestreckte Anordnung des Kraftübertragungsmittels. Richtungsangaben, die auf die Längsachse bezogen sind, gelten für flexible Kraftübertragungsmittel dabei nicht nur für die gestreckte Anordnung, sondern auch in entsprechend abgewandelter Weise, bei gebogener Anordnung des flexiblen Kraftübertragungsmittel. Z. B. sind Bewegungen des Kraftübertragungsmittels entlang der Längsachse allgemeiner als Hin- und Herbewegungen des Kraftübertragungsmittels innerhalb des Instrumentenschafts zu verstehen, also auch bei gebogenem Verlauf.

Als Betätigungsmittel eines chirurgischen Instruments können händisch betätigbare Griffteile, Aktuatoren oder alternativ robotische Betätigungseinheiten zum Einsatz kommen.

Bezüglich der elektrisch leitenden Materialien und elektrisch isolierenden Materialien der verschiedenen Komponenten des erfindungsgemäßen Maulteilwerkzeugs wird folgendes bemerkt: Bei den elektrisch leitenden Materialien der elektrisch angeschlossenen Komponenten wie den vorgenannten Branchen, Ansteuerungslaschen und Kraftübertragungsmittel, sowie sämtliche nachfolgend angeführte elektrisch angeschlossene Komponenten, kann es sich um dasselbe elektrisch leitende Material oder um unterschiedliche elektrisch leitende Materialien handeln, die der Fachmann nach den jeweiligen Anforderungen entsprechend auszuwählen weiß. Entsprechendes gilt in ähnlicher Weise für die elektrisch isolierenden Materialien des vorgenannten Maulteilkerns und sämtlicher nachfolgend angeführter elektrisch isolierender Komponenten. D. h. die verschiedenen elektrisch isolierender Komponenten können das gleiche elektrisch isolierende Material aufweisen, oder es können je nach Anforderung an die jeweilige Komponente unterschiedliche elektrisch isolierende Materialien verwendet werden.

Nach einer weiteren Ausführungsform kann das erfindungsgemäße Maulteilwerkzeug eine Gabelhalterung aufweisen, die die Lagerung der Branchen an dem Maulteilkern gegen Lösen sichert. Die Gabelhalterung besteht aus elektrisch isolierendem Material und weist proximalseitig zumindest einen Rohrabschnitt auf, der zur Anordnung auf zumindest einem Rohrabschnitt des Maulteilkerns vorgesehen und ausgebildet ist. Distalseitig weist die Gabelhalterung zwei Halteabschnitte auf, die dazu ausgebildet sind, den Maulteilkern um die Aussparungen formschlüssig zu fassen, und den Eingriff der Ansteuerungslaschen mit den Betätigungsabschnitten der Branchen sowie deren gelenkige Lagerung an den Lagerungsabschnitten in den jeweiligen Aussparungen zu sichern

Zur formschlüssigen Fassung des Maulteilkerns begrenzen die Halteabschnitte eine Innenkontur, die entsprechend der Außenkontur des Maulteilkerns mit den darin angeordneten Abschnitten der Branchen und Ansteuerungslaschen in einem Bereich um die Aussparungen geformt ist. Der Formschluss wirkt über die Berührung der zu den Gelenkachsen orthogonalen Innenflächen der beiden Halteabschnitte mit den nach außen weisenden Flächen des Maulteilkerns, der Branchen und der Ansteuerungslaschen in dem Bereich um die Aussparungen. Auf diese Weise sperren die Halteabschnitte eine Lösebewegung der Branchen und Ansteuerungslaschen aus den Aussparungen in eine von der Längsachse wegweisenden, zur Gelenkachse parallelen Richtung.

Nach einer weiteren Ausführungsform des erfindungsgemäßen Maulteilwerkzeugs werden die Gelenkachsen der Branchen in den Aussparungen des Maulteilkerns jeweils durch einen in einer Gelenkbuchse drehbar gelagerten Gelenkzapfen gebildet. Vorzugsweise sind dabei die Gelenkbuchsen in den Aussparungen und die Gelenkzapfen an den Branchen ausgebildet, sodass die Gelenkbuchsen in die Kernwand hineinragen und die Branchen vorteilhaft einstückig mit den Gelenkzapfen gefertigt werden können. Alternativ ist es aber auch möglich, dass die Gelenkzapfen als separate Achselemente mit den Branchen verbunden werden, oder dass die Gelenkzapfen an den Aussparungen, als Vorsprung an der Kernwand, und die Gelenkbuchsen in den Branchen ausgebildet sind. Auch dabei können die Gelenkzapfen einstückig mit dem Maulteilkern oder als separate Achselemente vorliegen.

Dabei sind die Aussparungen vorzugsweise mitsamt den Gelenkbuchsen (alternativ Gelenkzapfen) in Bezug auf die Längsachse rotationssymmetrisch an dem Maulteilkern auf gegenüberliegenden Seiten der Kernwand ausgebildet, sodass beide Branchen identisch gefertigt sein können. Die beiden Gelenkachsen, die orthogonal zu der Längssachse verlaufen, sind radial davon beabstandet, sodass sie bei der rotationssymmetrischen Anordnung der Aussparungen nicht miteinander fluchten und keine Verbindung zwischen den Aussparungen herstellen können.

Alternativ dazu ist es auch möglich, dass eine der Aussparungen eine Gelenkbuchse und die andere Aussparung einen Gelenkzapfen zur Bildung der Gelenkachsen aufweist, sodass von den Branchen eine einen Gelenkzapfen und die zweite eine Gelenkbuchse aufweist. Diese Ausführung bedingt zwar eine komplexere Fertigung, es kann aber bei einem Werkzeug mit unterschiedlichen Branchen vorteilhaft sein, wenn jede Branche nur in der passenden zugeordneten Aussparung angeordnet werden kann.

Nach einer weiteren Ausführungsform des erfindungsgemäßen Maulteilwerkzeugs ist vorgesehen, dass der Eingriff der Ansteuerungslasche mit dem Betätigungsabschnitt der Branche durch einen in einer Lagerungsöffnung aufgenommenen Betätigungszapfen bereitgestellt wird, der eine Betätigungsachse definiert, die von der Gelenkachse beabstandet und parallel dazu ist. Vorzugsweise ist auch hier der Betätigungszapfen an dem Betätigungsabschnitt der Branche und die Lagerungsöffnung in der Ansteuerungslasche ausgebildet. Auch hier ist eine einstückige Fertigung des Betätigungszapfens mit der Branche bevorzugt, allerdings ist auch die Verwendung eines separaten Achselements als Betätigungszapfen möglich, der mit der Branche verbunden wird. Alternativ dazu kann die Lagerungsöffnung in dem Betätigungsabschnitt der Branche und der Betätigungszapfen an der Ansteuerungslasche ausgebildet sein.

Der Eingriff des Betätigungszapfens in der Lagerungsöffnung sorgt für die Bewegung zum Öffnen und Schließen der Maulteilabschnitte der Branchen und gleichzeitig für die Übertragung des elektrischen Potentials. Der Abstand der Betätigungsachse von der Gelenkachse bestimmt das Drehmoment zum Drehen der Branche um die Gelenkachse und wird für ein günstiges Hebelarmverhältnis zu dem Maulteilabschnitt möglichst groß gewählt. Eine Platzierung der Gelenkachse in der Aussparung möglichst am Rand des Maulteilkerns ermöglicht eine Maximierung des Abstands des Betätigungszapfen am Betätigungsabschnitt von der Gelenkachse innerhalb des zur Verfügung stehenden Bauraums.

Daher können in einer bevorzugten Ausführungsform des erfindungsgemäßen Maulteilwerkzeugs der Betätigungszapfen an dem Betätigungsabschnitt und der Gelenkzapfen an dem Lagerungsabschnitt der jeweiligen Branche in entgegengesetzte Richtungen weisend angeordnet sein.

Nach noch einer weiteren Ausführungsform des erfindungsgemäßen Maulteilwerkzeugs kann jede Branche einteilig aus dem elektrisch leitenden Material ohne Isolationsschichten oder Isolatorabschnitte gefertigt sein. Dabei weist der Maulteilabschnitt zumindest eine Funktionsfläche zum Präparieren, Fassen oder Schneiden und zur Elektrokoagulation auf. Und der Betätigungsabschnitt ist zum flächigen Kontakt mit einem flächigen Kontaktabschnitt der Ansteuerungslasche aus einem elektrisch leitenden Material ausgebildet. Dabei sind die jeweilige Kontaktflächen des Betätigungsabschnitts und des Kontaktabschnitts orthogonal zur Gelenkachse, sodass sie von den Halteabschnitten der Gabelhalterung aneinandergedrückt werden und der elektrische Übergangswiderstand besonders gering ist.

Ferner ist vorgesehen, dass das Kraftübertragungsmittel eines erfindungsgemäßen Maulteilwerkzeugs nach einer weiteren Ausführungsform zur Verbindung mit den Ansteuerungslaschen eine Hülse aufweist. Dabei ist das Kraftübertragungsmittel in einen ersten Abschnitt, der ein distaler, unisolierter Endabschnitt ist, und in einen zweiten, umfänglich isolierten Abschnitt unterteilt, der sich an den distalen Endabschnitt anschließt und proximalseitig von einem dritten, umfänglich isolierten Abschnitt des Kraftübertragungsmittels im Durchmesser abgesetzt ist. Die Hülse ist an dem zweiten Abschnitt angeordnet, sodass die Hülse durch die umfängliche Isolation elektrisch von dem Kraftübertragungsmittel getrennt ist, und erstreckt sich bis zu dem dritten Abschnitt, wobei die Hülse mechanisch mit dem Kraftübertragungsmittel verbunden ist. Dabei ist eine erste Ansteuerungslasche mit dem distalen, unisolierten Endabschnitt elektrisch und mechanisch verbunden. Die zweite Ansteuerungslasche, die länger ist als die erste Ansteuerungslasche, ist mit der Hülse elektrisch und mechanisch verbunden.

Nach noch einer weiteren Ausführungsform des erfindungsgemäßen Maulteilwerkzeugs kann der zumindest eine Rohrabschnitt des Maulteilkerns eine Längsbohrung aufweisen, in die sich das Kraftübertragungsmittel längsbeweglich erstreckt, sodass der distale Endabschnitt des Kraftübertragungsmittels in der Längsbohrung liegt.

In einer Weiterbildung davon weist der Maulteilkern eines erfindungsgemäßen Maulteilwerkzeugs für jede Ansteuerungslasche jeweils ein Führungsprofil auf, in der jeweils eine der Ansteuerungslaschen geführt und von dem Kraftübertragungsmittel in eine Richtung bewegt wird, die in einer Ebene mit der Längsachse liegt. Dabei ist ein erstes Führungsprofil zur Führung der ersten, mit dem distalen Endabschnitt des Kraftübertragungsmittels verbundenen Ansteuerungslasche ausgebildet. Dazu erstreckt sich das erste Führungsprofil von dem Betätigungsschwenkbereich einer der Aussparungen entlang des zumindest einen Rohrabschnitts. Das erste Führungsprofil endet vor einem proximalen Ende des zumindest einen Rohrabschnitts und mündet in die Längsbohrung. Das zweite Führungsprofil, das zur Führung der zweiten Ansteuerungslasche ausgebildet ist, die mit der Hülse verbunden ist, erstreckt sich von dem Betätigungsschwenkbereich der anderen Aussparung bis zu dem proximalen Ende des zumindest einen Rohrabschnitts.

Nach noch einer weiteren Ausführungsform weist das erfindungsgemäße Maulteilwerkzeug eine Schaftverbinderhülse auf, die mit dem Maulteilkern proximalseitig verbunden ist und zumindest ein Schaftverbindungselement zur Verbindung mit dem Instrumentenschaft aufweist.

In einer bevorzugten Weiterbildung dieses erfindungsgemäßen Maulteilwerkzeugs kann die Schaftverbinderhülse zur Anordnung auf einem proximalen Rohrabschnitt der Gabelhalterung ausgebildet sein, der auf einem proximalen Rohrabschnitt des Maulteilkerns angeordnet wird. Dabei weisen die Schaftverbinderhülse und die proximalen Rohrabschnitte der Gabelhalterung und des Maulteilkerns jeweils eine radial ausgerichtete, miteinander fluchtende Arretieröffnung auf, in der ein Arretierelement angeordnet ist, das die Schaftverbinderhülse und die Gabelhalterung mit dem Maulteilkern verbindet.

Weitere Ausführungsformen des erfindungsgemäßen Maulteilwerkzeugs beziehen sich auf Befestigungsvarianten der Gabelhalterung: So können die zwei Halteabschnitte der Gabelhalterung in einer Ausführungsform jeweils eine Öffnung aufweisen, die bezüglich Form und Abmessungen mit einem Kragen korrespondieren, der für lange Kriechstrecken an dem Maulteilkern benachbart zu der jeweiligen Aussparung, vorzugsweise distalseitig benachbart zu der Aussparung, ausgebildet ist. Damit sich die Halteabschnitte beim Aufschieben auf den Maulteilkern über die Kragen aufspreizen können, bis sie mit den Öffnungen an den Kragen einschnappen, sind die Halteabschnitte in radialer Richtung zur Längsachse L elastisch verformbar. Dazu kann beispielsweise das elektrisch isolierende Material der Gabelhalterung zumindest im Bereich der Halteabschnitte durch ein elastisch verformbares Kunststoffmaterial bereitgestellt werden und/oder jeder Halteabschnitt einen in der Wandstärke reduzierten Abschnitt aufweisen. Alternativ dazu, bei Verwendung eines nicht elastischen Materials wie z. B. Keramik, kann die Gabelhalterung aus zwei identischen Halterungsschalen zusammengesetzt werden, bzw. längs in zwei Halterungsschalen mit jeweils einem der Halteabschnitte geteilt sein. Die beiden den Maulteilkern formschlüssig umschließenden Halterungsschalen können proximalseitig von einer Hülse, beispielsweise der Schaftverbinderhülse, zusammengehalten werden. Der Spalt zwischen den beiden Halterungsschalen ist elektrisch unkritisch, da er in Längsrichtung quer zu den relevanten Kriechstrecken verläuft.

Um die zwei Halterungsschalen auch distalseitig zusammenzuhalten, bzw. die elastisch verformbaren Halteabschnitte einer einstückigen Gabelhalterung zu sichern, kann die Gabelhalterung in weiteren Ausführungsformen an den zwei Halteabschnitten durch zumindest ein zylindrisches Verbindungselement oder stoffschlüssig durch eine (Kunststoff-) Schweiß- oder Klebeverbindung mit dem Maulteilkern verbunden sein. Eine weitere alternative oder zusätzliche Ausführungsform bezieht sich auf eine stoffschlüssige Verbindung des zumindest einen Rohrabschnitts der Gabelhalterung mit dem Maulteilkern durch eine (Kunststoff-)Schweiß- oder Klebeverbindung.

Eine Sicherung durch Verbindungselement(e) kann vorteilhaft lösbar gestaltet sein, sodass das Maulteilwerkzeug nach einem Einsatz vollständig demontiert und nach einer Reinigung und Desinfektion wiederverwendet werden kann. Die stoffschlüssigen Verbindungen zwischen Gabelhalterung und Maulteilkern sorgen vorteilhaft für weitgehende Abdichtung der Aussparungen. Kriechstrecken zwischen den Ansteuerungslaschen werden dadurch weithingehend verhindert.

Die distalseitige Sicherung der Gabelhalterung an den zwei Halteabschnitten mit einem oder zwei zylindrischen Verbindungselement(en) erfolgt vorzugsweise an den Kragen, die die Halteabschnitte mit ihrer Öffnung umgreifen. Die Kragen begrenzen dann entweder eine durchgehende Bohrung für ein durchgehendes zylindrisches Verbindungselement, wie beispielsweise einen Niet, oder jeweils eine Sackbohrung für zwei einzelne Verbindungselemente, wie beispielsweise Schrauben.

Da ein durchgehendes Verbindungselement mit der Bildung einer Kriechstrecke verbunden ist, kann zusätzlich eine Abdichtung vorgesehen sein, um das Eindringen von leitenden Flüssigkeiten zu unterbinden. Eine Sicherung mit separaten Verbindungselementen wie Schrauben kommt ohne durchgehende Bohrung aus und vermeidet daher die Bildung einer solchen Kriechstrecke, auch ohne Abdichtung. Außerdem ermöglichen Schrauben vorteilhaft, die Haltekraft der Halteabschnitte zu verstärken und ein Anzugsmoment zu wählen, um den seitlichen Halt der Branchen zu verbessern, was insbesondere bei Scherenwerkzeugen wichtig ist, deren Maulteilabschnitte für eine saubere Trennung von Gewebe seitlich eng geführt werden müssen.

Dabei kann ferner in einer weiteren Ausführungsform vorgesehen sein, dass die eingesetzten Verbindungselemente kopfseitig mit einer Außenfläche der Halteabschnitte abschließen, um einen für das Maulteilwerkzeug vorgegebenen Außendurchmesser nicht zu überschreiten. Dazu kann die Öffnung in den Halteabschnitten einen abgesenkten Absatz aufweisen, an dem ein Kopf des Verbindungselements innerhalb der Öffnung anliegt. Zusätzlich oder alternativ dazu kann der Kopf des Verbindungselements spanend bearbeitet, z. B. überschliffen werden, um bündig mit der Außenfläche der Gabelhalterung abzuschließen. So werden seitliche Vorsprünge an dem Maulteilwerkzeug vermieden, die bei Einsatz im Patienten zu einem Hängenbleiben oder Verletzungen des Gewebes führen könnten.

Nach noch einer weiteren Ausführungsform des erfindungsgemäßen Maulteilwerkzeugs kann ein Schwenkbereich des Maulteilabschnitts jeder Branche in Bezug zu der Längsachse durch eine Begrenzungswand jeder Aussparung begrenzt werden, wobei ein Öffnungswinkel jedes Maulteilabschnitts in Bezug zu der Längsachse in einem Bereich liegt, der sich von 0 ° ( für geschlossene Maulteilabschnitte) bis zumindest 15 ° und höchstens 45 °, bevorzugt bis 30 ° erstreckt, die sich zu einem Gesamtöffnungswinkel zwischen beiden Maulteilabschnitten zu zumindest 30 °, maximal 90 °, bevorzugt 60 ° summieren. Der bevorzugte Gesamtöffnungswinkel von 60 ° ist für die meisten Anwendungen vollkommen ausreichend und vermeidet die mit einem größeren Öffnungswinkel verbundenen Nachteile, die in einem erhöhten Bauraumbedarf bei schlechterer Kraftübertragung bestehen.

Noch weitere Ausführungsformen des erfindungsgemäßen Maulteilwerkzeugs beziehen sich auf die in der Ansteuerungslasche ausgebildete Lagerungsöffnung für den Betätigungszapfen, der an dem Betätigungsabschnitt der Branche vorliegt. Nach einer Ausführungsform kann die Lagerungsöffnung in einer zur Längsachse orthogonalen Richtung länglich ausgeführt sein und parallele Seitenflächen aufweisen, deren Abstand dem Durchmesser des Betätigungszapfens entspricht. Diese längliche Lagerungsöffnung ist dazu ausgebildet, eine Ausgleichsbewegung des Betätigungszapfens in der Lagerungsöffnung in die zur Längsachse orthogonale Richtung zuzulassen. Die Ausgleichbewegung ist erforderlich, wenn die Ansteuerungslasche in ihrer Längsrichtung in einer Ebene mit Längsachse bewegt wird, da der Betätigungszapfen einer Kreisbahn um die Gelenkachse folgt.

Alternativ zu einer länglichen Lagerungsöffnung für eine Ausgleichsbewegung des Betätigungszapfens kann der Maulteilkern dazu ausgebildet sein, eine Ausgleichsbewegung der Ansteuerungslasche in einer zur Längsachse orthogonalen Richtung zuzulassen. Dazu kann vorzugsweise die Ansteuerungslasche in der zur Längsachse orthogonalen Richtung elastisch verformbar ausgebildet sein, um ein zusätzliches Gelenk an der Verbindung mit dem Kraftübertragungsmittel zu vermeiden. Für die Ausgleichsbewegung der Ansteuerungslasche kann das Führungsprofil mit entsprechendem Spiel dimensioniert werden. Dann ist es möglich, dass die Lagerungsöffnung zumindest teilweise zylindrisch mit einem Durchmesser ausgebildet ist, der einem Durchmesser des Betätigungszapfens entspricht. Hierbei wird bei der Kraftübertragung in Längsrichtung eine hohe Pressung vermieden, wie sie bei der Linienberührung des Betätigungszapfens in der länglichen Lagerungsöffnung entsteht. Bei einer teilweise zylindrischen Lagerungsbohrung ist die Zylinderform auf einer von der Gelenkachse wegweisenden Seite unterbrochen, d. h. die Lagerungsöffnung dort offen, um Platz zu sparen und die Betätigungsachse möglichst weit von der Gelenkachse beabstandet platzieren zu können.

Ein bipolares chirurgisches Instrument als weiterer erfindungsgemäßer Gegenstand weist ein Betätigungsmittel, einen Instrumentenschaft und ein Maulteilwerkzeug mit zwei schwenkbar gelagerten Branchen und einem Kraftübertragungsmittel auf. Die Branchen, die ein elektrisch leitendes Material aufweisen, sind an einem distalen Ende des Instrumentenschafts angeordnet. Das Kraftübertragungsmittel erstreckt sich entlang der Längsachse beweglich durch den Instrumentenschaft und ist mit dem Betätigungsmittel gekoppelt, das an einem proximalen Ende des Instrumentenschafts angeordnet ist. Erfindungsgemäß ist das Maulteilwerkzeug des bipolaren chirurgischen Instruments ein erfindungsgemäßes bipolares chirurgisches Maulteilwerkzeug nach zumindest einer der vorbeschriebenen Ausführungsformen.

In einer Ausführungsform kann das Betätigungsmittel des bipolaren chirurgischen Instruments ein händisch betätigbarer Griff sein, und in einer anderen Ausführungsform eine robotisch betätigbare Handhabe.

Ein weiterer erfindungsgemäßer Gegenstand ist ein robotisches chirurgisches Instrumentensystem, das zumindest eine Steuerungseinheit, einen elektrochirurgischen Generator und einen Roboterarm aufweist, der mit der Steuerungseinheit verbunden ist und ein erfindungsgemäßes bipolares chirurgisches Instrument aufweist, das mit dem elektrochirurgischen Generator verbunden ist.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Dabei zeigen:
- Fig. 1: eine perspektivische Ansicht eines Maulteilwerkzeugs nach einer erfindungsgemäßen Ausführungsform,
- Fig. 2: eine Seitenansicht eines erfindungsgemäßen bipolaren chirurgischen Instruments mit einem erfindungsgemäßen Maulteilwerkzeug,
- Fig. 3: eine perspektivische Ansicht des Maulteilkerns eines erfindungsgemäßen Maulteilwerkzeugs von einer ersten Seite,
- Fig. 4: eine perspektivische Ansicht des Maulteilkerns aus Fig. 3 von der anderen Seite,
- Fig. 5: eine perspektivische Ansicht des Maulteilkerns aus Fig. 3 mit einer Branche und dem distalen Ende des Kraftübertragungsmittels,
- Fig. 6: eine Querschnittansicht durch das Maulteilwerkzeug aus Fig. 1 mit einer zur Längsachse L orthogonalen Schnittebene durch die Gelenkachsen A, A',
- Fig. 7: eine perspektivische Ansicht des Maulteilkerns aus Fig. 3 mit einer Branche in Schließstellung, einer ersten Ansteuerungslasche und dem distalen Ende des Kraftübertragungsmittels,
- Fig. 8: eine perspektivische Ansicht entsprechend Fig. 7 mit der Branche in Offenstellung,
- Fig. 9: eine perspektivische Teilexplosionsansicht des Maulteilwerkzeug aus Fig. 1,
- Fig. 10: eine perspektivische Detailansicht des Maulteilkerns mit einer ersten Ansteuerungslasche nach einer alternativen Ausführungsform eines erfindungsgemäßen Maulteilwerkzeugs,
- Fig. 11: eine perspektivische Ansicht des Maulteilkerns aus Fig. 4 mit zwei Branchen, einer zweiten Ansteuerungslasche und des Kraftübertragungsmittels mit Hülse,
- Fig. 12: eine perspektivische Ansicht entsprechend Fig. 11 mit Gabelhalterung,
- Fig. 13: eine perspektivische Ansicht entsprechend Fig. 12 mit Schaftverbinderhülse,
- Fig. 14: eine Längsschnittansicht durch das Maulteilwerkzeug aus Fig. 13 mit einer zur Gelenkachsen A, A' parallelen Schnittebene durch die Längsachse und
- Fig. 15: eine perspektivische Ansicht eines robotischen chirurgischen Instrumentensystems mit einem erfindungsgemäßen bipolaren chirurgischen Instrument.

Die Erfindung betrifft ein Maulteilwerkzeug für bipolare chirurgische Instrumente mit zwei gegeneinander bewegbaren Branchen wie beidseitig öffnende Zangen- und Scheren-Instrumente. Ferner betrifft die Erfindung ein mit einem entsprechenden Maulteilwerkzeug ausgestattetes bipolares chirurgisches Instrument. Die Maulteilmechanik mit dem speziellen, bifunktionellen Maulteilkern aus elektrisch isolierendem, d. h. nichtleitenden, Material dient gleichzeitig zur elektrischen Trennung der Branchen und zu deren mechanischer Lagerung. Dies sorgt dafür, dass keine zusätzlichen Isolatoren an den Branchen benötigt werden.

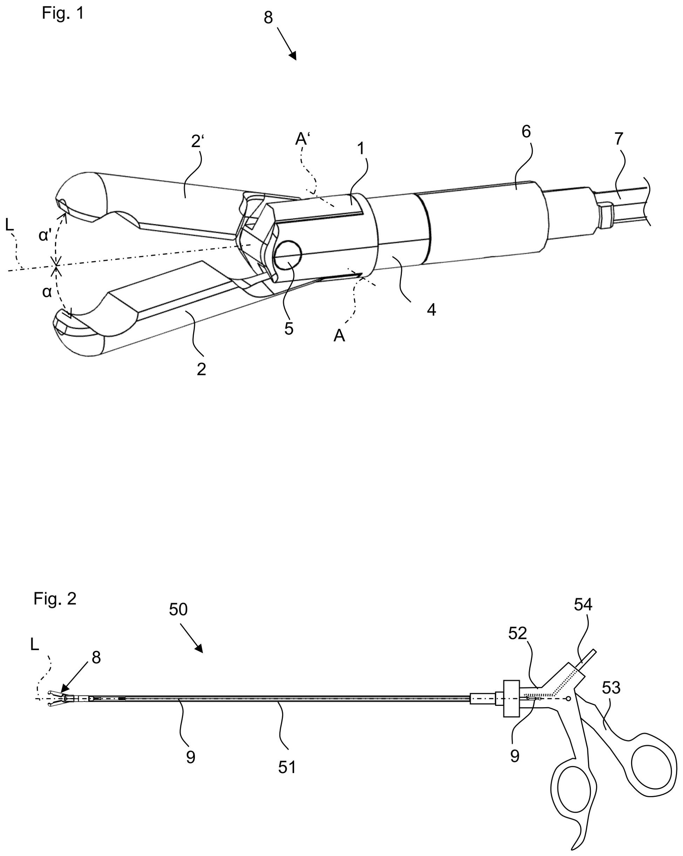

Das in den Figuren 1 bis 14 dargestellte Maulteilwerkzeug 8 ist als Einsatz für ein bipolares chirurgisches Instrument 50 ausgebildet, wie beispielhaft in Fig. 2 gezeigt ist. Das Maulteilwerkzeug 8, dessen beide Branchen 2, 2' schwenkbar gelagert sind und ein elektrisch leitendes Material aufweisen, ist an dem distalen Ende eines Instrumentenschafts 51 angeordnet. Ein Kraftübertragungsmittel 9 des Maulteilwerkzeugs 8 steht mit den Branchen 2, 2' in Wirkverbindung und erstreckt sich entlang einer gemeinsamen Längsachse L längsbeweglich durch den Instrumentenschaft 51, der an seinem proximalen Ende mit einem Betätigungsmittel 52 verbunden ist, bei der es sich im dargestellten Beispiel um einen händisch betätigbaren Griff 52 handelt. Dieser hat ein bewegliches Griffteil 53, das mit dem Kraftübertragungsmittel 9 zum Öffnen und Schließen der Branchen 2, 2' mechanischen verbunden ist, was durch die gestrichelte Linie angedeutet wird. Ferner ist der Griff 52 mit einer Anschlussbuchse 54 ausgestattet, um das bipolare chirurgische Instrument 50 zur Versorgung mit elektrischer Energie an einem Generator (nicht dargestellt) anzuschließen. Durch den Griff 52 wird eine elektrische Verbindung der Anschlussbuchse 54 mit dem Kraftübertragungsmittel 9 hergestellt, was durch die gepunkteten Linien angedeutet wird, um die Branchen 2, 2' bei Anlegen von Strom als Elektroden zur Elektrokoagulation nutzen zu können. Alternativ zu dem beispielhaften händisch betätigbaren Griff, ist es auch möglich, dass ein erfindungsgemäßes bipolares chirurgisches Instrument eine robotische Betätigungseinheit aufweist, die mittels eines Eingabegeräts gesteuert wird.

Die beiden Branchen 2, 2' des Maulteilwerkzeug 8 werden an einem Maulteilkern 1 gelagert, der aus einem elektrisch isolierenden Material besteht. Hierfür wird vorzugsweise ein Kunststoff- oder Keramikmaterial mit hohem CTI-Wert gewählt. Beispielsweise kann der Maulteilkern 1 aus einem Kunststoff mit hohem CTI-Wert wie Polyvinylidenfluorid PVDF (CTI-Wert 600) gefertigt, z. B. gefräst oder in Serie im Spritzguss hergestellt werden. Vorteilhaft ist es aber auch möglich, den Maulteilkern 1 in Serie aus Keramik herzustellen, und dadurch beste mechanische, chemische und elektrische Eigenschaften zu erhalten.

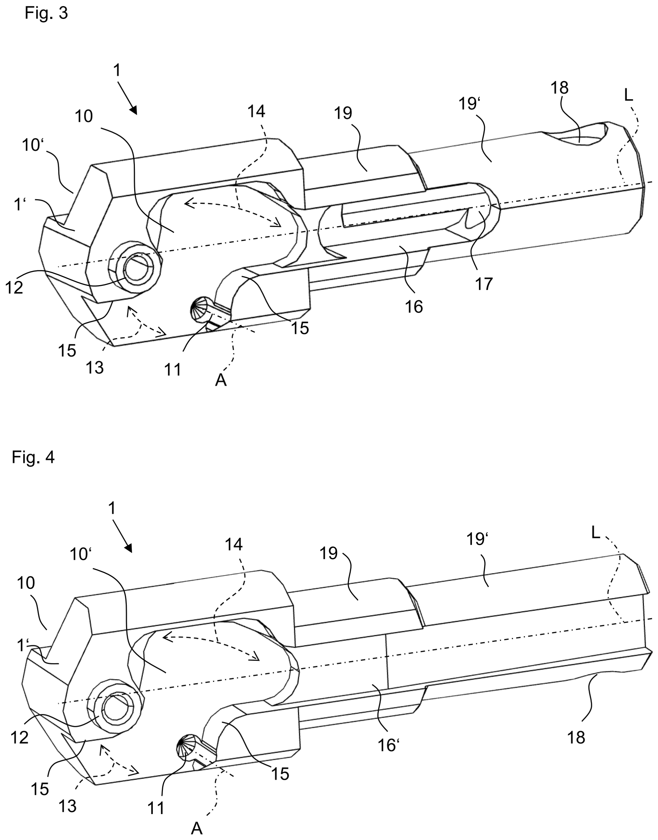

In Fig. 3 und 4 ist der Maulteilkern 1 von beiden Seiten gezeigt, die zwei durch eine Kernwand 1' voneinander getrennten Aussparungen 10, 10' zur Lagerung der Branchen 2, 2' aufweist. Fig. 3 zeigt eine erste Aussparung 10 am Maulteilkern 1 zur drehbaren Lagerung einer ersten Branche 2 um eine erste Gelenkachse A. In Fig. 4 ist die Seite des Maulteilkerns 1 mit der zweiten Aussparung 10' zu sehen, zur drehbaren Lagerung einer zweiten Branche 2' um eine zweite Gelenkachse A' ausgebildet ist.

Die beiden Aussparungen 10, 10' sind dabei rotationssymmetrisch in Bezug auf die Längsachse L auf gegenüberliegenden Seiten der Kernwand 1' ausgebildet, sodass die beiden Branchen 2, 2' im dargestellten Beispiel identisch ausgeformt sind. Und weil die Branchen 2, 2' durch den Maulteilkern 1 elektrisch voneinander komplett isoliert sind, können die Branchen 2, 2' einstückig aus einem elektrisch leitenden Material ohne zusätzliche Isolatoren gefertigt werden.

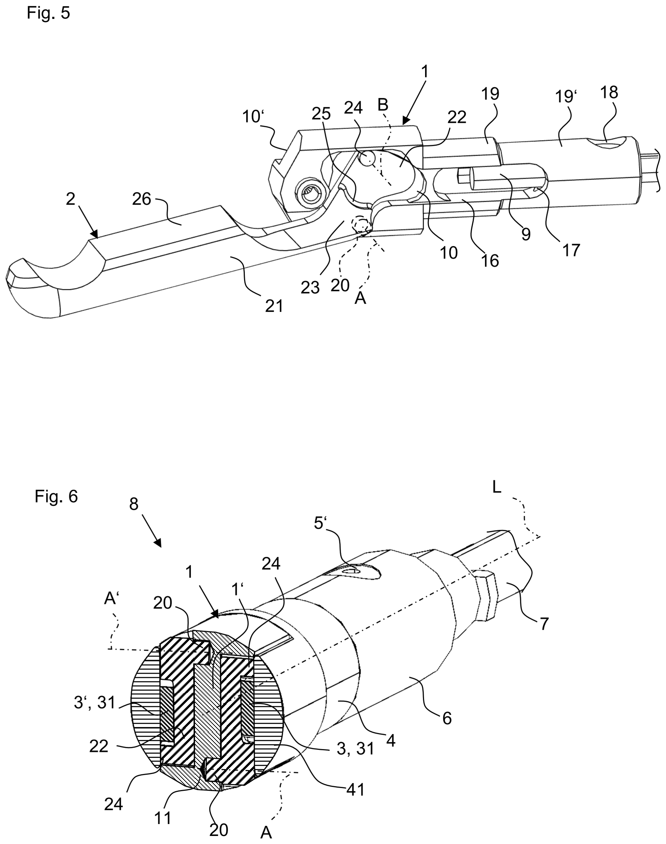

Für die beiden Gelenkachsen A, A', durch die jede Aussparung 10, 10' in einen Austrittsschwenkbereich 13 und einen Betätigungsschwenkbereich 14 unterteilt wird, ist in der Kernwand 1' jeweils eine Gelenkbuchse 11 ausgebildet. Diese durchdringt nicht die Kernwand 1', sodass keine Verbindung zwischen den beiden Aussparungen 10, 10' entsteht. Die beiden Gelenkachsen A, A' verlaufen orthogonal zu der Längssachse L und sind in radialer Richtung bezogen auf die Längsachse L davon beabstandet, was auch in Fig. 6 zu erkennen ist. In der Gelenkbuchse 11 ist ein Gelenkzapfen 20 drehbar aufgenommen, den jede Branche 2, 2' aufweist. In Fig. 5 ist der in der Darstellung verdeckte Gelenkzapfen 20 der ersten Branche 2 gestrichelt dargestellt. Dort ist zu erkennen, dass der Gelenkzapfen 20 bzw. die dadurch gebildete Gelenkachse A an einem abgewinkelten Lagerungsabschnitt 23 vorliegt. Dieser trennt einen Maulteilabschnitt 21 der Branche 2, der sich durch den Austrittsschwenkbereich 13 der Aussparung 10 erstreckt, von einem Betätigungsabschnitt 22 der Branche 2, der in dem Betätigungsschwenkbereich 14 der Aussparung 10 angeordnet ist. Der Maulteilabschnitt 21 jeder Branche 2, 2' hat zumindest eine Funktionsfläche 26, einerseits zum Fassen, Präparieren oder Schneiden und andererseits zur Elektrokoagulation. Entsprechendes gilt auch für die drehbare Lagerung der zweiten Branche 2' in der zweiten Aussparung 10'.

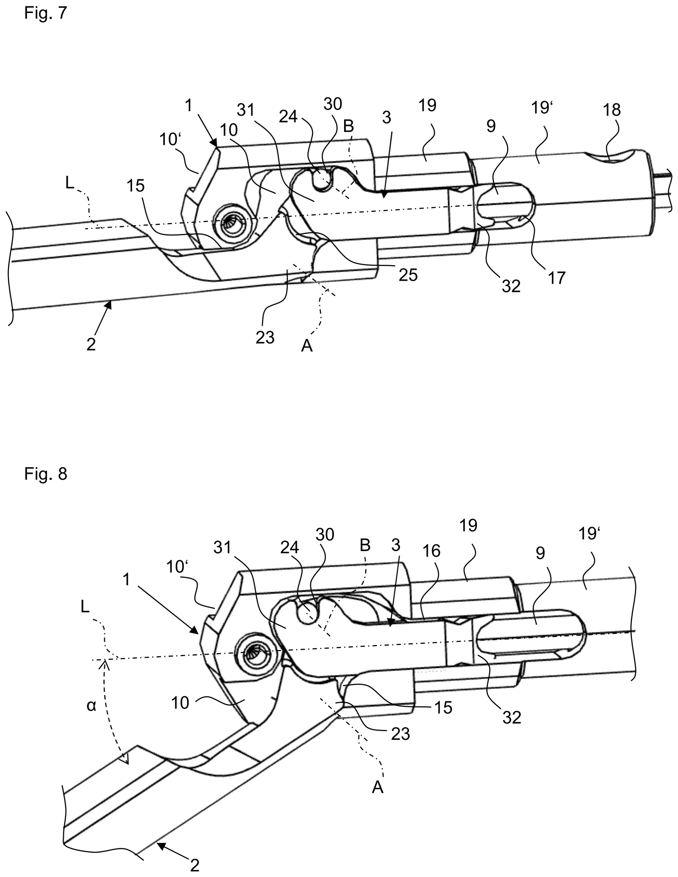

Der Betätigungsabschnitt 22 jeder Branche 2, 2' dient zum Eingriff einer mit dem Kraftübertragungsmittel 9 verbundenen Ansteuerungslasche 3, 3' für die mechanische und elektrische Ansteuerung der Branchen 2, 2', zu sehen in Fig. 7 bis 11 und 14. D. h. die Ansteuerungslasche 3, 3' sorgt einerseits für die mechanische Übertragung der Bewegung dem Kraftübertragungsmittel 9 auf die Branche 2, 2' und andererseits für die Übertragung elektrischer Energie. Daher sind auch die Ansteuerungslaschen 3, 3', vorzugsweise einstückig, aus einem elektrisch leitenden Material gefertigt.

Zum mechanischen Eingriff der Ansteuerungslasche 3, 3' mit dem Betätigungsabschnitt 22 ist an dem Betätigungsabschnitt 22 ein Betätigungszapfen 24 in entgegengesetzter Richtung zu dem Gelenkzapfen 20 ausgebildet, wie Fig. 5 und 6 zeigen. Dieser Betätigungszapfen 24 ist in einer Lagerungsöffnung 30 der Ansteuerungslasche 3, 3' aufgenommen. So definiert der Betätigungszapfen 24 eine Betätigungsachse B, die von der Gelenkachse A, A' beabstandet ist und parallel dazu verläuft. Der elektrische Eingriff der Ansteuerungslasche 3, 3' mit dem Betätigungsabschnitt 22 erfolgt über einen flächigen Kontakt, wozu ein Kontaktabschnitt 31 der Ansteuerungslasche 3, 3' in einem Bereich um die Lagerungsöffnung 30 flächig ausgebildet ist. Entsprechend ist auch der Betätigungsabschnitt 22 in einem Bereich um den Gelenkzapfen 20 flächig ausgebildet, sodass die Kontaktfläche für einen geringen elektrischen Übergangs- bzw. Kontaktwiderstand möglichst groß ist. Dabei sind die jeweiligen Kontaktflächen des Betätigungsabschnitts 22 und des Kontaktabschnitts 31 orthogonal zur Gelenkachse A, A'.

Dieser Betätigungszapfen 24 wird von der Ansteuerungslasche 3, 3' bewegt, um den Maulteilabschnitt 21 zu öffnen und zu schließen. Je weiter Gelenk- und Betätigungszapfen 20, 24 und damit Gelenk- und Betätigungsachse A, A'; B auseinander liegen, desto besser ist das Hebelverhältnis zum Maulteilabschnitt 21. Anders ausgedrückt, bestimmt der Abstand der Betätigungsachse B, dem mechanischen Eingriffspunkt der Ansteuerungslasche 3, 3', von der Gelenkachse A, A' den Hebelarm für das Drehmoment, mit dem die Branche 2, 2' um die Gelenkachse A, A' bewegt wird. Daher ist der Abstand des Betätigungszapfens 24 von dem Gelenkzapfen 20 größtmöglich gewählt, indem der Betätigungszapfen 24 am Rand des Betätigungsabschnitts 22, maximal entfernt von der Gelenkachse A, A' angeordnet ist. Mit größerem Hebelarm zwischen Betätigungszapfen 24 und Gelenkzapfen 20 kann in der Anwendung das Öffnen und Schließen der Maulteilabschnitte 21 filigraner angesteuert werden, und bei gleicher Kraft auf das Kraftübertragungsmittel 9 kann eine höhere Schließkraft zwischen den Maulteilabschnitten 21 erreicht werden.

Durch die flächige Form des Betätigungsabschnitts 22 und den Abstand zwischen der Gelenk A, A' und Betätigungsachse B werden elektrischer Kontakt und mechanischer Eingriff optimiert. Daher werden Form und Abmessungen des Betätigungsabschnitts 22 mit der Form und den Abmessungen des Betätigungsschwenkbereichs 14 der Aussparung 10, 10' so abgestimmt, dass der Betätigungsschwenkbereich 14 eine Bewegung des Betätigungsabschnitts 22 um die Gelenkachse A, A' innerhalb eines Winkelbereichs zum Öffnen und Schließen des Maulteilabschnitts 21 derselben Branche 2, 2' zulässt. Dabei ist zu berücksichtigen, dass der für die Aussparung 10, 10' zur Verfügung stehende Bauraum bei einem Maulteilwerkzeug 8 für ein chirurgisches Instrument 50 häufig auf wenige Millimeter im Durchmesser begrenzt ist. Daher sind Abweichungen der Betätigungsabschnitte und der Aussparungen eines erfindungsgemäßen Maulteilwerkzeugs 8 bezüglich Form und Abmessungen vom dargestellten Beispiel möglich und hängen nicht nur von dem zur Verfügung stehenden Bauraum, sondern auch von der Funktion der Branchen und deren Öffnungswinkel ab, wobei angestrebt, im vorhandenen Bauraum einen möglichst großen Hebelarm zwischen Betätigungsachse B und Gelenkachse A, A' zu erreichen

Die Aussparung 10, 10' ist vorliegend mit einer Begrenzungswand 15 so ausgeformt, dass jede der beiden (symmetrischen) Branchen 2, 2' in einem Öffnungswinkel α, α' im Bereich von 0 ° bei geschlossenen Maulteilabschnitten 21 bis jeweils 30 ° in Bezug auf die Längsachse L aufklappen kann. Damit ergibt sich summiert ein maximaler Öffnungswinkel zwischen beiden Maulteilabschnitten 21 von 60 °. Dieser ist für die meisten Maulteilwerkzeuge ausreichend bzw. üblich. Größere Winkel lassen sich zwar realisieren, erfordern allerdings mehr Bauraum und sind mit schlechterer Kraftübertragung verbunden, ohne in den meisten Anwendung einen Mehrwert zu erzielen.

Zur Sicherung sowohl der drehbaren Lagerung der Branchen 2, 2' in den Aussparungen 10, 10' als auch des Eingriffs der Ansteuerungslaschen 3, 3' mit den Branchen 2, 2' weist das Maulteilwerkzeug 8 eine Gabelhalterung 4 auf, zu sehen in Fig. 1, 6, 9, 12 bis 14.

Die Gabelhalterung 4 besteht aus elektrisch isolierendem Material, die distalseitig zwei Halteabschnitte 41, 41' aufweist, die sich von einem Rohrabschnitt 44 erstrecken, der von einem proximalseitigen Rohrabschnitt 43 abgesetzt ist. Die Halteabschnitte 41, 41' sind rotationssymmetrisch zum formschlüssigen Fassen des Maulteilkerns 1 um die Aussparungen 10, 10' ausgebildet. Die Halteabschnitte 41, 41' haben dazu eine zur Kernwand 1' parallele Innenfläche, mit der die Halteabschnitte 41, 41' die Branchen 2, 2' und Ansteuerungslaschen 3, 3' in den Aussparungen 10, 10' hält. Da dabei die Ansteuerungslaschen 3, 3' mit ihren Kontaktabschnitten 31 flächig an die Betätigungsabschnitte 22 der Branchen 2, 2' gedrückt werden, ist der elektrische Übergangswiderstand vorteilhaft besonders gering. Distalseitig schließen die Halteabschnitte 41, 41' bündig mit dem Maulteilkern 1 ab.

Die formschlüssige Anordnung der Gabelhalterung 4 auf dem Maulteilkern 1 wird durch die Rohrabschnitte 43, 44 ergänzt, die auf korrespondierend geformten Rohrabschnitten 19, 19' des Maulteilkerns 1 angeordnet werden. D. h.; dass ein Innendurchmesser des abgesetzten Rohrabschnitts 44 der Gabelhalterung 4 einem Außendurchmesser des abgesetzten Rohrabschnitts 19 des Maulteilkerns 1 und ein Innendurchmesser des proximalseitigen Rohrabschnitts 43 der Gabelhalterung 4 einem Außendurchmesser des proximalseitigen Rohrabschnitts 19' des Maulteilkerns 1 entsprechen. Außerdem sind die abgesetzten Abschnitte 44; 19 und die proximalen Abschnitte 43; 19' mit jeweils entsprechenden Längen ausgebildet.

Die Gabelhalterung 4 weist zur Bildung langer Kriechstrecken in jedem Halteabschnitt 41, 41' am freien, distalen Ende eine Öffnung 40 auf, die bei Anordnung der Gabelhalterung 4 an dem Maulteilkern 1 einen bezüglich Form und Abmessungen korrespondierenden Kragen 12 aufnimmt, der distalseitig benachbart zu der Aussparung 10, 10' an dem Maulteilkern 1 ausgebildet ist. Damit sich die Halteabschnitte 41, 41' beim Aufschieben der Gabelhalterung 4 auf den Maulteilkern 1 etwas aufspreizen lassen, bevor sie mit den Öffnungen 40 um den Kragen 12 einschnappen, ist die Gabelhalterung 4 aus einem Kunststoff gefertigt, der eine elastische Verformung der Halteabschnitte 41, 41' zulässt. Damit bildet die an dem Maulteilkern 1 formschlüssig angeordnete Gabelhalterung 4 schon ohne weitere Verbindungsmittel eine stabile Einheit und die sichert die Branchen 2, 2' gegen Lösen. Für die Öffnungsmechanik der Branchen 2, 2' mit dem Maulteilkern 1 und den Ansteuerungslaschen 3, 3' hat die Gabelhalterung 4 aber keine Relevanz.

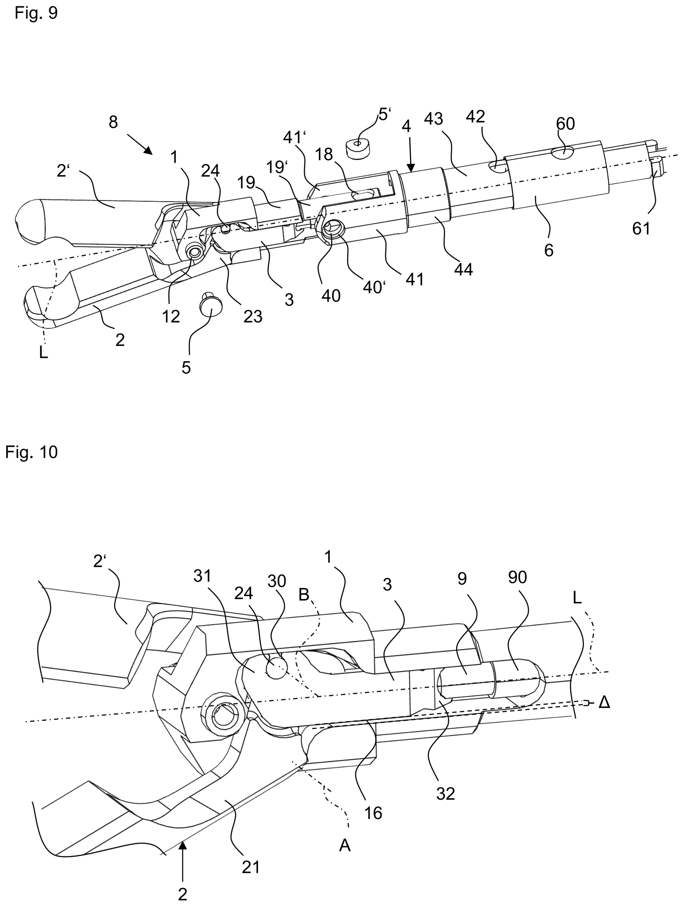

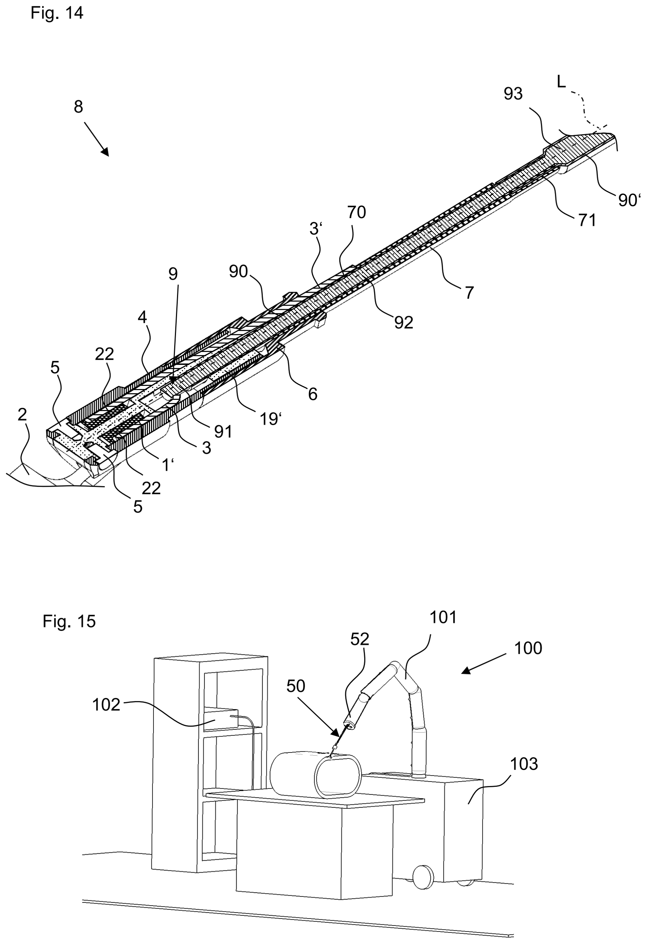

Um den seitlichen Halt der Branchen 2, 2' weiter zu verbessern, sind die Halteabschnitte 41, 41' jeweils mit einem zylindrischen Verbindungselement 5, hier eine Schraube, gesichert. Dies ist insbesondere bei Scherenwerkzeugen wichtig, deren Maulteilabschnitte 21 seitlich eng geführt werden müssen, damit das Gewebe nicht zwischen den Schneidkanten durchrutscht, sondern sauber getrennt wird. Die Schraube 5 wird durch die Öffnung 40 in den Kragen 12 eingefügt, sodass ein abgesetzter Kopf der Schraube 5 die Öffnung 40 überdeckt und die Halteabschnitte 41, 41' an dem Maulteilkern 1 fixiert. Damit die Schraube 5 kopfseitig mit der Außenfläche der Halteabschnitte 41, 41' abschließt, ist in der Öffnung 40 ein abgesenkter Absatz 40' ausgebildet, zu sehen in Fig. 9, 14. Zusätzlich oder alternativ kann der Kopf der Schraube 5 spanend bearbeitet z. B. überschliffen werden, um mit der Oberfläche des Halteabschnitts 41, 41' abzuschließen.

Ferner weist die Gabelhalterung 4 in dem proximalseitigen Rohrabschnitt 43 eine radial ausgerichtete Arretieröffnung 42 auf, die bei formschlüssiger Anordnung auf dem Maulteilkern 1 mit einer entsprechenden Arretieröffnung 18 im proximalseitigen Rohrabschnitt 19' des Maulteilkerns 1 fluchtet. Die Arretieröffnungen 18, 42 sind zur Aufnahme eines Arretierelements 5', hier Arretierbolzen 5', ausgebildet, der insbesondere zur Befestigung einer Schaftverbinderhülse 6 vorgesehen ist. Die Schaftverbinderhülse 6, die zur Anordnung auf dem proximalseitigen Rohrabschnitt 43 der Gabelhalterung 4 ausgebildet ist, weist entsprechend eine Arretieröffnung 60 auf, die bei bestimmungsgemäßer Anordnung mit den Arretieröffnungen 42, 18 der Gabelhalterung 4 und des Maulteilkerns 1 fluchtet. Außerdem weist die Schaftverbinderhülse 6 hier zwei Bajonettnocken 61 als Schaftverbindungselemente auf, die zur Verbindung mit einem Instrumentenschaft 51 dienen, welcher hierzu an seinem distalen Ende zwei Bajonettnuten (nicht abgebildet) aufweist. Dazu alternative Verbindungselemente zur (lösbaren) Verbindung eines erfindungsgemäßen Maulteilwerkzeugs 8 mittels einer Schaftverbinderhülse 6 an einem Instrumentenschaft 51 umfassen z. B. Schraub-, Steck- und Rastverbindungen.

Ferner kann ein erfindungsgemäßes Maulteilwerkzeug 8 eine Gabelhalterung aufweisen, die vom dargestellten Beispiel abweicht. Beispielsweise könnte die Gabelhalterung aus zwei identischen Halterungsschalen aus einem nicht-elastisch verformbaren Kunststoffmaterial oder auch Keramik, bestehen. Die Halbschalen, die jeweils einen der Halteabschnitte aufweisen, umschließen den Maulteilkern 1 in entsprechender Weise formschlüssig und werden distalseitig von den zylindrischen Verbindungselementen 5 und proximalseitig von einer Hülse, beispielsweise der Schaftverbinderhülse 6 zusammengehalten. Der zwischen den Halterungsschalen verbleibende Spalt wäre elektrisch unkritisch, weil er in Längsrichtung, also quer zu den relevanten Kriechstrecken zwischen den Ansteuerungslaschen 3, 3' bzw. den Betätigungsabschnitten 22 der Branchen 2, 2' verläuft.

Eine weitere Alternative sieht ein durchgehendes zylindrisches Verbindungselement (z. B. Niet) anstelle der beiden Schrauben 5 vor. Da sich hierfür eine Durchtrittsbohrung zwischen den Kragen 12 quer zur Längsachse L durch den Maulteilkern 1 erstreckt, kann zur Verringerung des Kriechstreckenrisikos das durchgehende Verbindungselement aus einem elektrisch nichtleitenden Material gewählt werden und/oder ein Abstand des Kragens - und damit der Durchtrittsbohrung - von der Aussparung vergrößert werden.

Als Ergänzung oder weitere Alternative ist eine stoffschlüssige Verbindung der Halteabschnitte 41, 41' und/oder der Rohrabschnitte 43, 44 der Gabelhalterung 4 mit dem Maulteilkern 1, z. B. durch Kunststoffschweißen oder Kleben etc., denkbar. Denn durch den Stoffschluss kann jegliche Kriechstrecke zwischen den Ansteuerungslaschen bzw. den Betätigungsabschnitten komplett unterbunden werden, da keine Flüssigkeit in das Maulteilwerkzeug eindringen kann. Bei rein formschlüssiger Befestigung sorgt eine Presspassung bzw. das Anzugsmoment des Verbindungselement dafür, das eine Spaltentstehung zwischen den Halteabschnitten und dem Maulteilkern und damit das Eindringen von Flüssigkeit verhindert bzw. minimiert werden.

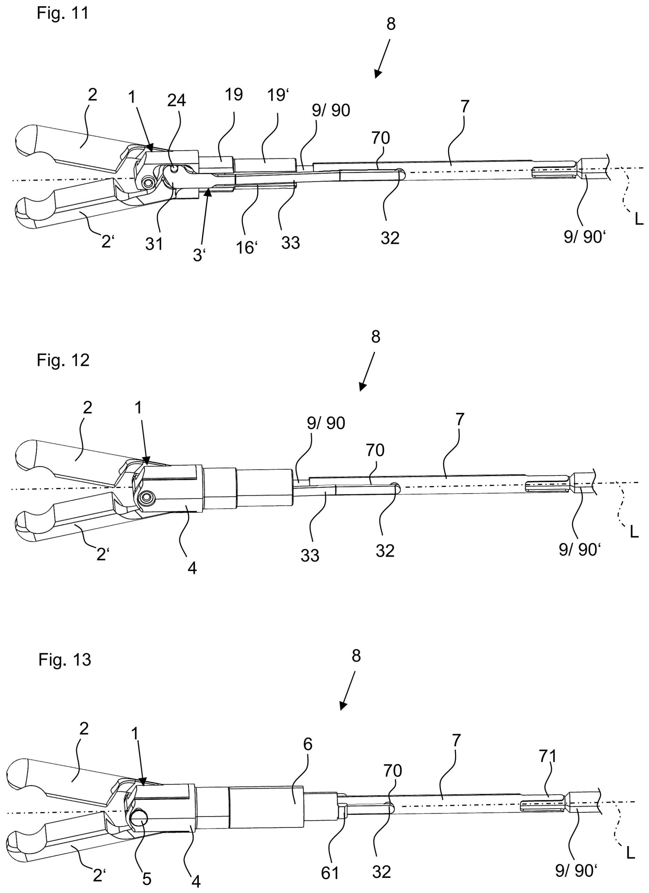

Zur elektrisch getrennten Verbindung jeder Ansteuerungslasche 3, 3' mit dem Kraftübertragungsmittel 9, das aus einem elektrischen leitenden Material besteht, weist das Maulteilwerkzeug 8 eine Hülse 7 als zweiten elektrischen Leiter auf, die in Fig. 1, 6 teilweise und insbesondere in Fig. 11 bis 14 ganz abgebildet ist. Dabei ist die erste Ansteuerungslasche 3 mechanisch und elektrisch mit dem Kraftübertragungsmittel 9 an einem ersten, distalen Endabschnitt 91 verbunden, der unisoliert ist. Die zweite Ansteuerungslasche 3 ist mechanisch und elektrisch mit der Hülse 7 verbunden, die an einem zweiten Abschnitt 92 des Kraftübertragungsmittels 9 proximalseitig zu dem Maulteilkern 1 angeordnet ist. Die zweite Ansteuerungslasche 3' ist daher länger ausgeführt als die erste Ansteuerungslasche 3. Der zweite Abschnitt 92, der sich an den distalen Endabschnitt 91 anschließt, ist mit einer Isolatorschicht 90, z. B. durch einen Schrumpfschlauch, ummantelt. Durch die Isolatorschicht 90 und den Abstand zu dem distalen Endabschnitt 91 ist die Hülse 7 elektrisch von dem Kraftübertragungsmittel 9 isoliert. Die Hülse 7 und das Kraftübertragungsmittel 9 sorgen damit für die elektrische Leitung durch den Instrumentenschaft 51 zwischen den Ansteuerungslaschen 3, 3' und dem Betätigungsmittel 51 mit dem elektrischen Anschluss 54.

Mechanisch ist die Hülse 7, die sich proximalseitig bis zu einem dritten Abschnitt 93 des Kraftübertragungsmittels 9 erstreckt, der von dem zweiten Abschnitt 92 abgesetzt ist, mit dem Kraftübertragungsmittel 9 verbunden, sodass die Bewegung des Kraftübertragungsmittels 9 in Richtung der Längsachse L zum Öffnen und Schließen der Maulteilabschnitte 21 über beide Ansteuerungslaschen 3, 3' übertragen wird. Im dargestellten Beispiel weist die Hülse 7 eine distale Längsöffnung 70 auf, die zur Aufnahme eines proximalen Verbindungsabschnitts 32 der zweiten Ansteuerungslasche 3' dimensioniert ist.

Das Kraftübertragungsmittel 9 ragt mit ihrem distalen Endabschnitt 91 längsbeweglich in eine Längsbohrung 17 des Maulteilkerns 1, die sich durch den proximalseitigen Rohrabschnitt 19' in den abgesetzten Rohrabschnitt 19' erstreckt. Der distale Endabschnitt 91, mit dem die erste Ansteuerungslasche 3 verbunden wird, befindet sich so innerhalb der Längsbohrung 17 im Bereich der Rohrabschnitte 19, 19'. Damit die erste Ansteuerungslasche 3 sich von der Aussparung 10 in die Längsbohrung 17 erstrecken kann, ist ein Durchbruch erforderlich. Dazu ist an dem Maulteilkern 1 ein erstes Führungsprofil 16 zur Führung der ersten Ansteuerungslasche 3 ausgebildet, das sich von dem Betätigungsschwenkbereich 14 der ersten Aussparung 10 entlang dem abgesetzten Rohrabschnitt 19 erstreckt und im Übergangsbereich zu dem proximalen Rohrabschnitt 19' in die Längsbohrung 17 mündet, wie in Fig. 3, 7, 8, 10 und 14 erkennbar ist. Für die zweite Ansteuerungslasche 3' erstreckt sich das zweite Führungsprofil 16' von dem Betätigungsschwenkbereich 14 der zweiten Aussparung 10' entlang den beiden Rohrabschnitten 19, 19', um die zweite Ansteuerungslasche 3' in Richtung der Hülse 7 zu führen (Fig. 4, 11, 14). Die Führungsrichtungen beider Führungsprofile 16, 16' liegen in einer Ebene mit der Längsachse L, sodass die Ansteuerungslaschen 3, 3' in etwa parallel zur Längsachse L bewegt werden.

Der von der jeweiligen Ansteuerungslasche 3, 3' bewegte Betätigungszapfen 24 folgt einer Kreisbahn um die Gelenkachse A, A' und vollzieht daher beim Öffnen und Schließen der Maulteilabschnitte 21 gegenüber der parallel zur Längsachse L bewegten Ansteuerungslasche 3, 3' auch einen Bewegungsanteil in einer zur Längsachse L orthogonalen Richtung. Darum ist die Lagerungsöffnung 30 in der Ansteuerungslasche 3, 3' in einer zur Längsachse L orthogonalen Richtung länglich ausgeführt, sodass der Betätigungszapfen 24 auf- und abgleiten kann, wie in Fig. 7 und 8 erkennbar ist. Die längliche Lagerungsöffnung 30, die nach außen, d. h. auf einer von der Längsachse L wegweisenden Seite geöffnet ist, hat parallelen Seitenflächen, deren Abstand dem Durchmesser des Betätigungszapfens 24 entspricht. Die Kraftübertragung in Längsrichtung erfolgt dabei zwischen Seitenfläche der länglichen Lagerungsöffnung 30 und Zylinderfläche des Betätigungszapfen 24.

Um die mit einer solchen Linienberührung verbundene erhöhte Pressung zu vermeiden, kann die Lagerungsöffnung 30 der Ansteuerungslasche 3, 3' in einer alternativen Ausführung, die in Fig. 10 verdeutlicht wird, wie der Betätigungszapfen 24 zylindrisch ausgeführt sein, sodass eine flächige, umfängliche Kraftübertragung ermöglicht ist. Auch hierbei kann die Lagerungsöffnung 30 nach außen, d. h. auf einer von der Längsachse L wegweisenden Seite geöffnet sein, sodass die Zylinderform der Lagerungsöffnung 30 teilweise unterbrochen ist, um den Betätigungszapfen 24 in dem zur Verfügung stehenden Bauraum möglichst entfernt von der Gelenkachse A, A' platzieren zu können. Da in einer zumindest teilweise zylindrischen Lagerungsöffnung 30, deren Durchmesser dem Durchmesser des Betätigungszapfens 24 entspricht, keine Ausgleichsbewegung des Betätigungszapfens 24 in der zur Längsachse L orthogonalen Richtung möglich ist, ist hierbei das Führungsprofil 16, 16' in dem Maulteilkern 1 mit seitlichem Spiel für die Ansteuerungslasche 3, 3' ausgebildet. So kann die Ansteuerungslasche 3, 3' mit dem Betätigungszapfen 24 die Ausgleichsbewegung in der zur Längsachse L orthogonalen Richtung ausführen. Dabei ist das Ausmaß dieser Ausgleichsbewegung gegenüber der Länge der Ansteuerungslasche 3, 3' so gering, dass diese sich elastisch vorformen kann und kein Gelenk zur Verbindung mit dem Kraftübertragungsmittel 9 benötigt. Aufgrund des mit Spiel ausgeführten Führungsprofils 16, 16' entsteht ein in Fig. 10 ersichtlicher Spalt Δ an der in der Abbildung unteren Kante der Ansteuerungslasche 3, 3', wenn sich der Betätigungszapfen 24 an dem in der Abbildung oberen Umkehrpunkt der Kreisbahn um die Gelenkachse A, A' befindet. Sind die Maulteilabschnitte 21 hingegen maximal geöffnet oder geschlossen (nicht abgebildet), befindet sich der Betätigungszapfen 24 an den bezogen auf die Abbildung aus Fig. 10 tiefsten Endpunkten seiner Kreisbahn, sodass die Ansteuerungslasche 3, 3' nach unten bewegt wird und der Spalt in dem Führungsprofil 16, 16' an der Oberkante der Ansteuerungslasche 3, 3' entsteht.

Fig. 15 zeigt ein robotisches chirurgisches Instrumentensystem 100 mit einem bipolaren chirurgischen Instrument 50. Dieses befindet sich an einem Ende eines Roboterarms 101, der das Betätigungsmittel 52 des bipolaren chirurgischen Instruments 50 bereitstellt. Der Roboterarm 101 ist mit einer Steuerungseinheit 102 verbunden und stellt die elektrische Verbindung zwischen dem elektrochirurgischen Generator 103 und dem bipolaren chirurgisches Instrument 50 bereit.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung stellt ein bipolares chirurgisches Maulteilwerkzeug 8 und ein damit geschaffenes bipolares chirurgisches Instrument 50 bereit. Das Maulteilwerkzeug 8 hat zwei schwenkbar gelagerte Branchen 2, 2', die ein elektrisch leitendes Material aufweisen, und ein Kraftübertragungsmittel 9, das mit den Branchen 2, 2' in Wirkverbindung steht, und ist dazu ausgebildet, durch Kopplung mit einem Instrumentenschaft 51 und mit einem Betätigungsmittel 52 das Instrument 50 bereitzustellen, wobei die Branchen 2, 2' distalseitig am Instrumentenschaft 51 angeordnet sind und das Kraftübertragungsmittel 9 in dem Instrumentenschaft 51 entlang einer Längsachse L beweglich angeordnet und mit dem Betätigungsmittel 52 proximalseitig am Instrumentenschaft 51 in Eingriff bringbar ist. Das Maulteilwerkzeug 8 hat einen Maulteilkern 1 aus einem elektrisch isolierenden Material und zwei durch eine Kernwand 1' voneinander getrennte Aussparungen 10, 10', die jeweils eine Gelenkachse A, A' bereitstellen, die die Aussparungen 10, 10' jeweils in einen Austritts- und einen Betätigungsschwenkbereich 13, 14 unterteilt. Dabei hat jede Branche 2, 2' zwischen einem Maulteil- und einem Betätigungsabschnitt 21, 22 einen Lagerungsabschnitt 23, an dem die Branche 2, 2' um die jeweilige Gelenkachse A, A' schwenkbar in der Aussparung 10, 10' gelagert ist, wobei sich der Maulteilabschnitt 21 durch den Austrittsschwenkbereich 13 aus der Aussparung 10, 10' erstreckt und der Betätigungsabschnitt 22 in dem Betätigungsschwenkbereich 14 der Aussparung 10, 10' angeordnet ist. Das Maulteilwerkzeug 8 hat zwei Ansteuerungslaschen 3, 3', die mit dem Kraftübertragungsmittel 9 verbunden sind und sich jeweils in den Betätigungsschwenkbereich 14 der Aussparungen 10, 10' erstrecken und dort mit dem Betätigungsabschnitt 22 der jeweiligen Branche 2, 2' in Eingriff stehen. Ferner wird ein robotisches chirurgisches Instrumentensystem offenbart.

### BEZUGSZEICHENLISTE

- 1: Maulteilkern
- 1': Kernwand
- 2, 2': Branchen
- 3, 3': Ansteuerungslasche
- 4: Gabelhalterung
- 5, 5': Zylindrisches Verbindungselement, Arretierelement
- 6: Schaftverbinderhülse
- 7: Hülse
- 8: Maulteilwerkzeug
- 9: Kraftübertragungsmittel
- 10, 10': Aussparung
- 11: Gelenkbuchse
- 12: Befestigungskragen
- 13: Austrittsschwenkbereich
- 14: Betätigungsschwenkbereich
- 15: Begrenzungswand
- 16, 16': Führungsprofil
- 17: Längsbohrung
- 18: Arretieröffnung
- 19, 19': Abgesetzter Rohrabschnitt, Proximaler Rohrabschnitt
- 20: Gelenkzapfen
- 21: Maulteilabschnitt
- 22: Betätigungsabschnitt
- 23: Lagerungsabschnitt
- 24: Betätigungszapfen
- 25: Begrenzungsabsatz
- 26: Funktionsfläche
- 30: Lagerungsöffnung für Betätigungszapfen
- 31: Kontaktabschnitt
- 32: Verbindungsabschnitt
- 33: Verlängerungsabschnitt
- 40, 40': Aufnahmeöffnung, abgesenkter Absatz
- 41, 41': Halteabschnitt
- 42: Arretieröffnung
- 43, 44: Proximaler Rohrabschnitt, abgesetzter Rohrabschnitt
- 50: Bipolares chirurgisches Instrument
- 51: Instrumentenschaft
- 52: Betätigungsmittel/Griff
- 53: Bewegliches Griffteil
- 54: Anschlussbuchse
- 60: Arretieröffnung
- 61: Schaftverbindungselement/Bajonettnocken
- 70: Distale Längsöffnung
- 71: Proximale Längslasche
- 90, 90': Isolatorschichten
- 91, 92, 93: Distaler Endabschnitt/erster Abschnitt, zweiter Abschnitt, dritter/abgesetzter Abschnitt
- 100: Robotisches chirurgisches Instrumentensystem
- 101: Roboterarm
- 102: Steuerungseinheit
- 103: Elektrochirurgischer Generator
- A, A': Gelenkachse
- B: Betätigungsachse
- L: Längsachse
- α, α': Öffnungswinkel
- Δ: Spalt

## Patentansprüche

1. Bipolares chirurgisches Maulteilwerkzeug (8), das zwei schwenkbar gelagerte Branchen (2, 2'), die ein elektrisch leitendes Material aufweisen, und ein Kraftübertragungsmittel (9) aufweist, das mit den Branchen (2, 2') in Wirkverbindung steht, wobei das Maulteilwerkzeug (8) dazu ausgebildet ist, durch Kopplung mit einem Instrumentenschaft (51) und mit einem Betätigungsmittel (52) ein bipolares chirurgisches Instrument (50) bereitzustellen, wobei die Branchen (2, 2') an einem distalen Ende des Instrumentenschafts (51) anordenbar sind und das Kraftübertragungsmittel (9) in dem Instrumentenschaft (51) entlang einer gemeinsamen Längsachse (L) beweglich anordenbar und mit dem Betätigungsmittel (52) an einem proximalen Ende des Instrumentenschafts (51) in Eingriff bringbar ist,
**dadurch gekennzeichnet, dass**
das Maulteilwerkzeug (8) einen Maulteilkern (1) aufweist, der aus einem elektrisch isolierenden Material besteht und zwei durch eine Kernwand (1') voneinander getrennte Aussparungen (10, 10') aufweist, die ausgebildet sind, jeweils eine Gelenkachse (A, A') bereitzustellen, die die Aussparungen (10, 10') jeweils in einen Austrittsschwenkbereich (13) und einen Betätigungsschwenkbereich (14) unterteilt,
wobei jede Branche (2, 2') zwischen einem Maulteilabschnitt (21) und einem Betätigungsabschnitt (22) einen Lagerungsabschnitt (23) aufweist, an dem die Branche (2, 2') um die jeweilige Gelenkachse (A, A') schwenkbar in der Aussparung (10, 10') gelagert ist, wobei sich der Maulteilabschnitt (21) durch den Austrittsschwenkbereich (13) aus der Aussparung (10, 10') erstreckt und der Betätigungsabschnitt (22) in dem Betätigungsschwenkbereich (14) der Aussparung (10, 10') angeordnet ist, und wobei das Maulteilwerkzeug (8) zwei Ansteuerungslaschen (3, 3') aufweist, die mit dem Kraftübertragungsmittel (9) verbunden sind und sich jeweils in den Betätigungsschwenkbereich (14) der Aussparungen (10, 10') erstrecken und dort mit dem Betätigungsabschnitt (22) der jeweiligen Branche (2, 2') in Eingriff stehen.

2. Maulteilwerkzeug (8) nach Anspruch 1,
**dadurch gekennzeichnet, dass**
das Maulteilwerkzeug (8) eine Gabelhalterung (4) aus elektrisch isolierendem Material aufweist, die proximalseitig zumindest einen Rohrabschnitt (43, 44) zur Anordnung auf zumindest einem Rohrabschnitt (19, 19') des Maulteilkerns (1) und distalseitig zwei Halteabschnitte (41, 41') aufweist, die dazu ausgebildet sind, den Maulteilkern (1) um die Aussparungen (10, 10') formschlüssig zu fassen.

3. Maulteilwerkzeug (8) nach Anspruch 1 oder 2,
**dadurch gekennzeichnet, dass**
die Gelenkachsen (A, A') der Branchen (2, 2') in den Aussparungen (10, 10') des Maulteilkerns (1) jeweils durch einen in einer Gelenkbuchse (11) drehbar gelagerten Gelenkzapfen (20) gebildet werden, wobei die Gelenkbuchsen (11) in den Aussparungen (10, 10') und die Gelenkzapfen (20) an den Branchen (2, 2') oder die Gelenkzapfen an den Aussparungen (10, 10') und die Gelenkbuchsen in den Branchen (2, 2') ausgebildet sind,
und wobei die Aussparungen (10, 10') in Bezug auf die Längsachse (L) rotationssymmetrisch an dem Maulteilkern (1) auf gegenüberliegenden Seiten der Kernwand (1') ausgebildet sind, und wobei die beiden Gelenkachsen (A, A') orthogonal zu der Längssachse (L) verlaufen und radial davon beabstandet sind.

4. Maulteilwerkzeug (8) nach zumindest einem der Ansprüche 1 bis 3,
**dadurch gekennzeichnet, dass**
der Eingriff der Ansteuerungslasche (3, 3') mit dem Betätigungsabschnitt (22) durch einen in einer Lagerungsöffnung (30) aufgenommenen Betätigungszapfen (24) bereitgestellt wird, der eine Betätigungsachse (B) definiert, die von der Gelenkachse (A, A') beabstandet und parallel dazu ist, wobei der Betätigungszapfen (24) an dem Betätigungsabschnitt (22) und die Lagerungsöffnung (30) in der Ansteuerungslasche (3, 3') oder die Lagerungsöffnung in dem Betätigungsabschnitt (22) und der Betätigungszapfen an der Ansteuerungslasche ausgebildet sind.

5. Maulteilwerkzeug (8) nach zumindest einem der Ansprüche 1 bis 4,
**dadurch gekennzeichnet, dass**
jede Branche (2, 2') einteilig aus dem elektrisch leitenden Material gefertigt ist, wobei der Maulteilabschnitt (21) zumindest eine Funktionsfläche (26) aufweist, und der Betätigungsabschnitt (22) zum flächigen Kontakt mit einem flächigen Kontaktabschnitt (31) der Ansteuerungslasche (3, 3') aus einem elektrisch leitenden Material ausgebildet ist, wobei jeweilige Kontaktflächen des Betätigungsabschnitts (22) und des Kontaktabschnitts (31) zur Gelenkachse (A, A') orthogonal sind.

6. Maulteilwerkzeug (8) nach zumindest einem der Ansprüche 1 bis 5,
**dadurch gekennzeichnet, dass**
das Kraftübertragungsmittel (9) zur Verbindung mit den Ansteuerungslaschen (3, 3') eine Hülse (7) aufweist und in einen ersten Abschnitt (91), der ein distaler, unisolierter Endabschnitt (91) ist, und in einen zweiten, umfänglich isolierten Abschnitt (92) unterteilt ist, der sich an den distalen Endabschnitt (91) anschließt und proximalseitig von einem dritten, umfänglich isolierten Abschnitt (93) des Kraftübertragungsmittels (9) abgesetzt ist, wobei die Hülse (7) an dem zweiten Abschnitt (92) angeordnet ist und sich bis zu dem dritten Abschnitt (93) erstreckt, und
eine erste Ansteuerungslasche (3) mit dem distalen, unisolierten Endabschnitt (91) elektrisch und mechanisch verbunden ist, und
eine zweite Ansteuerungslasche (3'), die länger ist als die erste Ansteuerungslasche (3), mit der Hülse (7) elektrisch und mechanisch verbunden ist.

7. Maulteilwerkzeug (8) nach zumindest einem der Ansprüche 2 bis 6,
**dadurch gekennzeichnet, dass**
der zumindest eine Rohrabschnitt (19, 19') des Maulteilkerns (1) eine Längsbohrung (17) aufweist, in die sich das Kraftübertragungsmittel (9) längsbeweglich erstreckt.

8. Maulteilwerkzeug (8) nach Anspruch 7,
**dadurch gekennzeichnet, dass**
der Maulteilkern (1) für jede Ansteuerungslasche (3, 3') jeweils ein Führungsprofil (16, 16') aufweist, in dem die jeweilige Ansteuerungslasche (3, 3') geführt in eine Richtung bewegbar ist, die in einer Ebene mit der Längsachse (L) liegt, wobei
ein erstes Führungsprofil (16), das zur Führung der ersten Ansteuerungslasche (3) ausgebildet ist, die mit dem distalen Endabschnitt (91) verbunden ist, sich von dem Betätigungsschwenkbereich (14) einer ersten Aussparung (10) entlang des zumindest einen Rohrabschnitts (19, 19') erstreckt und vor einem proximalen Ende des zumindest einen Rohrabschnitts (19, 19') endet und in die Längsbohrung (17) mündet, und
ein zweites Führungsprofil (16'), das zur Führung der zweiten Ansteuerungslasche (3') ausgebildet ist, die mit der Hülse (7) verbunden ist, sich von dem Betätigungsschwenkbereich (14) einer zweiten Aussparung (10') bis zu dem proximalen Ende des zumindest einen Rohrabschnitts (19, 19') erstreckt.

9. Maulteilwerkzeug (8) nach zumindest einem der Ansprüche 1 bis 8,
**dadurch gekennzeichnet, dass**
das Maulteilwerkzeug (8) eine Schaftverbinderhülse (6) aufweist, die mit dem Maulteilkern (1) proximalseitig verbunden ist und zumindest ein Schaftverbindungselement (61), das zur Verbindung mit dem Instrumentenschaft (51) ausgebildet ist, aufweist.

10. Maulteilwerkzeug (8) nach Anspruch 9,
**dadurch gekennzeichnet, dass**
die Schaftverbinderhülse (6) zur Anordnung auf einem proximalen Rohrabschnitt (43) der Gabelhalterung (4) ausgebildet ist, der auf einem proximalen Rohrabschnitt (19') des Maulteilkerns (1) angeordnet ist, wobei die Schaftverbinderhülse (6) und der proximale Rohrabschnitt (43) der Gabelhalterung (4) und der proximale Rohrabschnitt (19') des Maulteilkerns (1) jeweils eine radial ausgerichtete, miteinander fluchtende Arretieröffnung (60, 42, 18) aufweisen, in der ein Arretierelement (5') angeordnet ist, das die Schaftverbinderhülse (6) und die Gabelhalterung (4) und den Maulteilkern (1) verbindet.

11. Maulteilwerkzeug (8) nach zumindest einem der Ansprüche 2 bis 10,
**dadurch gekennzeichnet, dass**
die zwei Halteabschnitte (41, 41') der Gabelhalterung (4) jeweils eine Öffnung (40) aufweisen, die bezüglich Form und Abmessungen mit einem Kragen (12) korrespondiert, der an dem Maulteilkern (1) benachbart zu der jeweiligen Aussparung (10, 10') ausgebildet ist, wobei die Halteabschnitte (41, 41') elastisch verformbar sind, oder
die Gabelhalterung (4) längs in zwei Halterungsschalen geteilt ist, die jeweils einen der Halteabschnitte (41, 41') aufweisen und proximalseitig von einer Hülse zusammengehalten werden.

12. Maulteilwerkzeug (8) nach zumindest einem der Ansprüche 2 bis 11,
**dadurch gekennzeichnet, dass**
die Gabelhalterung (4) an den zwei Halteabschnitten (41, 41') durch zumindest ein zylindrisches Verbindungselement (5) mit dem Maulteilkern (1) verbunden ist, oder
die zwei Halteabschnitte (41, 41') und/oder der zumindest eine Rohrabschnitt (43, 44) durch eine Schweiß- oder Klebeverbindung stoffschlüssig mit dem Maulteilkern (1) verbunden sind/ist.

13. Maulteilwerkzeug (8) nach zumindest einem der Ansprüche 1 bis 12,
**dadurch gekennzeichnet, dass**
ein Schwenkbereich des Maulteilabschnitts (21) jeder Branche (2, 2') in Bezug zu der Längsachse (L) durch eine Begrenzungswand (15) jeder Aussparung (10, 10') begrenzt wird, wobei ein Öffnungswinkel (α, α') jedes Maulteilabschnitts (21) in Bezug zu der Längsachse (L) in einem Bereich liegt, der sich von 0 ° bis zumindest 15 ° und höchstens 45 °, bevorzugt bis 30 ° erstreckt.

14. Maulteilwerkzeug (8) nach zumindest einem der Ansprüche 4 bis 13,
**dadurch gekennzeichnet, dass**
die Lagerungsöffnung (30) für den Betätigungszapfen (24)
- eine in einer zur Längsachse (L) orthogonalen Richtung längliche Lagerungsöffnung (30) mit parallelen Seitenflächen ist, deren Abstand einem Durchmesser des Betätigungszapfens (24) entspricht, wobei die längliche Lagerungsöffnung (30) dazu ausgebildet ist, eine Ausgleichsbewegung des Betätigungszapfens (24) in der Lagerungsöffnung (30) in die zur Längsachse (L) orthogonale Richtung zuzulassen, oder
- eine zumindest teilweise zylindrische Lagerungsöffnung (30) ist, deren Durchmesser einem Durchmesser des Betätigungszapfens (24) entspricht, wobei der Maulteilkern (1) ausgebildet ist, eine Ausgleichsbewegung der Ansteuerungslasche (3, 3') in einer zur Längsachse (L) orthogonalen Richtung zuzulassen, wobei bevorzugt die Ansteuerungslasche (3, 3') in der zur Längsachse (L) orthogonalen Richtung elastisch verformbar ausgebildet ist.

15. Bipolares chirurgisches Instrument (50), das ein Betätigungsmittel (52), einen Instrumentenschaft (51) und ein Maulteilwerkzeug (8) mit zwei schwenkbar gelagerten Branchen (2, 2') und einem Kraftübertragungsmittel (9) aufweist, wobei die Branchen (2, 2'), die ein elektrisch leitendes Material aufweisen, an einem distalen Ende des Instrumentenschafts (51) angeordnet sind, und sich das Kraftübertragungsmittel (9) entlang der Längsachse (L) beweglich durch den Instrumentenschaft (51) erstreckt und mit dem Betätigungsmittel (52) gekoppelt ist, das an einem proximalen Ende des Instrumentenschafts (51) angeordnet ist,
**dadurch gekennzeichnet, dass**
das Maulteilwerkzeug (8) ein bipolares chirurgisches Maulteilwerkzeug (8) nach zumindest einem der Ansprüche 1 bis 14 ist.

16. Robotisches chirurgisches Instrumentensystem (100), das zumindest eine Steuerungseinheit (102), einen elektrochirurgischen Generator (103) und einen Roboterarm (101) aufweist, der mit der Steuerungseinheit (102) verbunden ist und ein bipolares chirurgisches Instrument (50) aufweist, das mit dem elektrochirurgischen Generator (103) verbunden ist,
**dadurch gekennzeichnet, dass**
das bipolare chirurgische Instrument (50) ein bipolares chirurgisches Instrument (50) nach Anspruch 15 ist.
